# EUROPEAN PATENT APPLICATION

(11) **EP 2 245 942 A1**
(43) Date of publication of application: **03.11.2010**
(21) Application number: 08867741.4
(22) Date of filing: 30.12.2008
(51) Int. Cl.: A23G 1/42, A23G 1/48, A23L 1/30

(54) **COMPOSITION CONTAINING CACAO AND SPIRULINA**

(30) Priority: 02.01.2008 ES 200800055
(71) Applicant: Yañez Soler, Armando José, 03204 Elche (Alicante) (ES); Muñoz Cerda, Antonio, 03600 Elda (Alicante) (ES)
(72) Inventor: Yañez Soler, Armando José, 03204 Elche (Alicante) (ES); Muñoz Cerda, Antonio, 03600 Elda (Alicante) (ES)
(74) Representative: Isern-Jara, Nuria
(86) International application number: PCT/ES2008/000818
(87) International publication number: WO 2009/083629

(57) **Abstract**

The invention relates to a composition containing cacao and spirulina, made exclusively from natural products. The composition also contains variable proportions of: royal jelly, turmeric, maca, quinoa, stevia, jojoba and argan oil. The composition can take the form of a bar, sweet or drink. The composition is a high-energy appetite suppressant.

## Description

### Object of the invention

The present invention refers to an energy composition comprising cacao and the microalgae Spirulina, for food and medicinal uses.

### Technical background

Food bars made of chocolate and energizing elements are well known. However, in the composition of said bars we find a large amount of chemically synthesized products which are used to help incorporate the proteins, vitamins and aminoacids needed.

Fitness drinks, or energy drinks, normally contain a large quantity of sugars and have vitamins incorporated. These vitamins are not assimilated by the human body.

Chocolate is used in this type of energy products or compositions because of its energy value.

Cacao (*Theobroma cacao*) is rich in a great number of essential minerals, including magnesium, potassium, sodium, calcium, phosphorus and zinc, which have an important role in the human body.

Also, it is a source of flavonoids, vitamins such as vitamin E, and some of the B vitamins.

However, one of the best known effects of cacao is that produced on the state of mind, behaviour and mental activity. Because of its high content in xantins, cacao stimulates the central nervous system, the heart muscle and is generally a muscle relaxant.

Chocolate is one of the most craved-for products, both because of the great feeling of well-being it provides and its exquisite taste.

The use of chocolate (cacao), combined with other foods having medicinal and food purposes is widely known in the state of the art. Next, we present the use of chocolate in several patents:
WO 98/02165 discloses a method and a composition for reducing appetite and the craving for carbohydrates by using the neurotransmitter precursors serotonin, dopamine, norepinephrine and histamin, which include the precursors tryptophane, phenylalanine, tyrosine and histidine. According to this reference, precursors are combined with each other and with xantins to achieve a synergic effect which allows to reduce advantageously the dose of precursors. The xantins, including theobromine, cafeine and cacao, can be obtained from natural sources used in foods such as cacao, tea, coffee and other similar sources.
Patent US no. 6051236 discloses a food composition for optimizing muscle performance during exercise training and for increasing muscle cell repair and recovery after exercise, wherein the composition includes a carrier which can be chocolate, oat, wheat, cacao butter, semidry fruits, lipids and combinations thereof, providing a semiliquid carrier for the food composition in dry powder form.

The patent US no. 5612074 discloses a nutrient-enriched food-bar containing various ingredients, including vitamins, magnesium, aminoacids, and so on, wherein this food bar can be covered with materials such as chocolate, peanuts, caramel, honey, carob, fruit and yoghurt.

On the other hand, the state of the art does not comprise any food and/or medicinal product made by combining cacao with the algae Spirulina.

The algae Spirulina (*Spirulina platensis*), whose name comes from the Latin word for "spiral or helix" for its physical configuration, is a blue-green microscopic algae which contains many essential nutrients, i.e. chlorophyll, phycocianin and beta-carotene, vitamin B12, minerals such as iron, selenium, zinc and copper, and essential fatty acids such as gamma-linolenic acid. It contains about 65% of proteins of plant origin, comprising the 8 essential aminoacids, plus 9 others.

The effect of all microalgal ingredients in combination is greater than the benefit provided by each of them individually.

| | | | | | |
|---|---|---|---|---|---|
| Proteins | 60-70% | Phycocyanin | 1,400 mg | Arginine | 430 mg |
| Carbohydrates | 15-25% | y-Linolenic | 100 mg | Aspartic ac. | 610 mg |
| Minerals | 7-13% | cis-Linolenic | 80 mg | Cistin | 60 mg |
| Lipids | 6-8% | Vitamin A | 23.000 I.U. | Glutamic ac. | 910 mg |
| Fibre | 8-10% | Beta-carotene | 14 mg | Glycine | 320 mg |
| Calcium | 70 mg | Vitamin E | 1 mg | Histidine | 100 mg |
| Phosphorus | 80 mg | Vitamin B1 | 0.35 mg | Isoleucine | 350 mg |
| Magnesium | 40 mg | Vitamin B2 | 0.40 mg | Leucine | 540 mg |
| Iron | 10 mg | Vitamin B3 | 1.40 mg | Lysine | 290 mg |
| Zinc | 300 mg | Vitamin B6 | 80 µg | Metionine | 40 mg |
| Copper | 120 µg | Vitamin B12 | 25 µg | Phenylalanine | 280 mg |
| Manganese | 500 µg | Folic acid | 1 µg | Proline | 270 mg |
| Cromium | 25 µg | Biotin | 0.5 µg | Serine | 320 mg |
| Sodium | 90 mg | Pantotenic ac. | 10 µg | Treonine | 320 mg |
| Potasium | 140 mg | Vitamin C | 0.5 mg | Tryptophane | 90 mg |
| Selenium | 10 µg | Vitamin K1 | 200 µg | Tyrosine | 300 mg |
| Germanium | 60 µg | Inositol | 6.4 mg | Valine | 400 mg |
| Chlorophyll | 100 mg | S.O.D. | 15.000 I.U. | Sulfolipids | 100 mg |
| Carotenoids | 37 mg | Alanite | 470 mg | | |

As can be seen from the above table with the components of Spirulina, this algae is a biological source of proteins, vitamins, minerals and other nutrients.

Spirulina contains about 65% proteins, which is a much larger amount than that found in pork (about 28%) or even in beef (about 22%), being one of the most balanced natural vitamin reservoirs.

The following vitamins may be found:
- Beta-carotene, or provitamin A, the overdose of which is not possible, can contribute substantially to the antioxidant activity of Spirulina, as can vitamin E.
- Vitamin B12: Spirulina is one of the few source plants of this vitamin.
- Among the group of vitamins B, thiamin (B1), riboflavin (B2), niacin (B3) and vitamin B6 reduce the effects of depression and anxiety disorders, having a beneficial effect on neuropsychiatric diseases.

Similarly, Spirulina is a great source of minerals such as iron, magnesium, selenium, zinc and copper, which are minerals easily absorbed by the body acting directly on nervous system tissues.

The essential or polyunsaturated fatty acids containing omega3 (gamma-linolenic, omega6-linolenic and omega9-oleic acids) have a beneficial anti-inflammatory effect and regulate blood levels of cholesterol.

Thanks to its content in phenylalanine, which is a natural appetite-suppressant aminoacid which acts directly on the hypothalamus, it controls mood changes and appetite.

Spirulina has one of the most powerful combinations of nutrients, such that it can even replace breast milk.

There are numerous studies on malnourished babies which have even lost their eyesight due to malnutrition, who have not only recovered their eyesight by ingesting the algae Spirulina, but who now enjoy excellent health.

Below we provide a description of the state of the art, in which we find references to the use of the algae Spirulina in the food sector.

WO 2007/094498: food composition composed of Spirulina and Chlorella. Food containing an antioxidant composition, in particular a natural food with effects on health and nutrition which contains Spirulina and Chlorella, and optionally kale, which can be ingested on a daily basis.

WO 2007/062274: algae and algal extract, as an additional diet composition. Additional composition for the human diet, comprising the biomass in powder form of the blue-green algae Spirulina, and astaxanthin. The composition is stable, effective and convenient for the health and well-being of both healthy people and people with illhealth.

None of the abovementioned references combines the blue-green algae Spirulina with cacao, this combination providing innumerable advantages not only for human health but also in the form of dietetic effects.

It is well known that cacao has a unique delicious taste, being one of the most craved-for foods by humans. However, Spirulina has a characteristic taste and odour which are not accepted by Europeans, and therefore its addition to cacao would transform it in a tasty and delicious product, also eliminating its odour.

Chocolate provides a large amount of energy. However, its action is quick and its beneficial effects are short-lived. The addition of Spirulina slows down the effect of chocolate, while maintaining an energy reservoir in muscles.

Normally, a large number of sugars and saturated fats are added to chocolate for its consumption, the ingestion of which is harmful to health. With Spirulina, said fats would not be required, resulting in a completely healthy combination, with a high content in nutrients and vitamins, and a low caloric content. Similarly, it would help to prevent oxidation processes, allowing the use of smaller amounts of preserving substances in chocolate.

Cacao is one of the most craved-for foods, especially by people following a diet. With the addition of Spirulina to chocolate, said compound may be eaten by people following a diet, and will even be beneficial to them because it can act as an appetite inhibitor when taken before meals.

Another population group which craves for chocolate are children. In addition, children often dislike vegetables and fruit, which are the foods providing the needed vitamins and proteins. The combination of Spirulina and chocolate may allow children to eat their favourite food, while also eating needed vitamins and proteins.

In addition to the endogenous ingredients of the composition, i.e. cacao and Spirulina, the present invention can contain 1 or more added ingredients acting in synergy to enhance their properties.

These natural additives comprise:
- Royal jelly: it is a fluid secretion made by bees from honey, nectar and water collected from their environment. It is energetic and stimulating, enhances the immune system and provides a higher resistance to periods of stress and fatigue.

When used in the composition of the present invention, royal jelly can be included in a proportion of about 0.5 to 5% of the final product.
- Turmeric: extract obtained from *Curcuma longa* L., herbaceous plant from India, China and Middle East, which favours the good functioning of the liver, reduces cholesterol levels and has anti-inflammatory properties. There are studies showing it eliminates cancerous substances and helps to control tumour growth.

When used in the composition of the present invention, turmeric can be present in a percentage of about 0.2 to 4% of the final product.
- Maca: extracted from the tuberous plant *Lepidium peruvianum,* native to the highlands and mountainous zones of the Peruvian Andes. It is a nutritional supplement which helps to enhance vitality and energy, having a globally beneficial effect on the body.

When used in the composition of the present invention, turmeric can be present in a percentage of about 0.5 to 10%.
- Quinoa (*Chenopodium quinoa*): cereal original from the Bolivian highland plateau, it has a high nutritional value because of its protein and aminoacid content. It does not have gluten, so it can be eaten by celiacs. It does not have cholesterol either.

When used in the composition of the present invention, quinoa can be present in a percentage of about 1 to 60% of the final product.
- Stevia or estevia (*Stevia rebaudiana*): used as sweetener. It is a shrub native to Paraguay and Brazil. It is a sweetener suitable for diabetics which has antioxidant properties.

When used in the composition of the present invention, stevia can be present in a percentage of about 0.2 to 2% of the final product.
- The Jojoba fruit (*Simmondsia chinensis*) or its extract, simmondsin, known for its effect as appetite inhibitor, eliminating the feelings of hunger.

When used in the composition of the present invention, jojoba can be present in a percentage of about 1 to 70%, or 0.2 to 10% of simmondsin of the final product.
- Argan oil (*Argania spinosa*): obtained from the fruit seeds of the tree species endemic to the southwest area of Morocco. It has a fine and pleasant taste, very aromatic and has dietetic properties. It has antioxidant effects and helps to reduce cholesterol levels.

When used in the composition of the present invention, the argan oil can be present in a percentage of about 1 to 15% of the final product.

All products used in the composition object of the present invention are commercially available products.

The composition of the invention can take the form of a special chocolate, sweet, energy bar, food supplement which can be carried in a pocket or bag; it can be included in a breakfast, lunch or dinner, o as an appetizer before a meal, as an energy drink provided at home or at school, in a camp or military installation, at sports facilities and during any type of political, religious or sporting event.

Importantly, products derived from the present application may be applied and used in cases of extreme need, as part of a survival kit, in aid programs during humanitarian disasters, for sportsmen, sailors or mountaineers, or for any person needing a high-energy food which is easily preserved and is of low weight and volume. Similarly, in cases in which living space is reduced, such as in airplanes, submarines, spaceships or satellites.

### EMBODIMENT

A preferred embodiment of the present invention consists of an energy composition **characterized in that** cacao and a bluegreen algae called Spirulina are mixed together, in a percentage of about 0.5 to 90% of Spirulina to cacao. Stevia can replace sugar, for use in diabetic people.

A particular embodiment of the present invention can consist of an energy composition comprising:

| Component | weight % relative to total |
|---|---|
| Cacao | 35 |
| Spirulina | 8 |
| Stevia | 2 |
| Argan oil | 5 |
| Maca | 10 |
| Quinoa | 40 |

In another particular embodiment of this same invention, of an energy composition made of cacao and Spirulina having an appetite inhibitory effect, it comprises:

| Component | weight % relative to total |
|---|---|
| Cacao | 45 |
| Spirulina | 30 |
| Simmondsin or Jojoba fruit | 15 |
| Sugar | 10 |

Another particular embodiment of the present invention of an energy composition made of cacao and Spirulina comprises:

| Component | weight % relative to total |
|---|---|
| Cacao | 35 |
| Spirulina | 10 |
| Royal jelly | 8 |
| Maca | 8 |
| Quinoa | 37 |
| Stevia | 2 |

A skilled person in the art will be aware that various example embodiments of the present invention have been described above in sufficient detail, and that the embodiments exemplify a wider set of embodiments which are comprised within the spirit and scope of the present invention. In these embodiments, many changes may be made without departing from the spirit and scope of the invention. Therefore, the following claims should at least be given the widest interpretation possible in view of the above exposure.

## Claims

1. Energy composition for food and medicinal use, **characterized in that** it comprises a mixture of cacao and the blue-green algae called Spirulina.

2. Energy composition according to claim 1, **characterized in that** Spirulina represents 0.5% of the total mixture comprised of cacao and Spirulina.

3. Energy composition according to claim 1, **characterized in that** the presence of Spirulina in the mixture with cacao comprises 90% of the total, forming a shell of Spirulina covered with cacao.

4. Energy composition according to claim 1, **characterized in that** the proportion of Spirulina in the mixture with cacao may be comprised between 0.5 and 90% of the total.

5. Energy composition according to claim 1, **characterized in that** natural products, such as natural fats, cereals, sweeteners and products with stimulating properties are added.

6. Energy composition according to claim 5, **characterized in that** the natural fats are formed from argan oil, the cereals comprise maca and quinoa, and the sweetener is stevia.

7. Energy composition according to claim 5, **characterized in that** the argan oil comprises 0.5 to 25% by weight relative to the total, the maca comprises 0.1 to 5% of the total, and the quinoa is comprised in a range of 1 to 55% of the total, and the stevia is comprised between 0.1 and 3% of the total.

8. Energy composition according to claim 5, **characterized in that** the products with stimulating properties can be any of the following natural products: royal jelly, turmeric, lipoic acid, policosanol and resveratrol.

9. Energy composition according to claim 1, **characterized in that** appetite inhibitory products are added.

10. Energy composition according to claim 9, **characterized in that** the appetite inhibitory effect is achieved by means of a mixture of the blue-green algae Spirulina, the cacao, the jojoba fruit or the simmondsin extract thereof.

11. Use of the energy composition according to claims 1 to 10 for preparing an energy bar.

12. Use of the energy composition according to claims 1 to 10 for preparing an energy drink by the dissolution of said composition in water, fruit juice or milk.

13. Use of the energy composition according to claims 1 to 10 for preparing an appetite inhibitory sweet.
